# EUROPEAN PATENT APPLICATION

(11) **EP 1 479 711 A1**
(43) Date of publication of application: **24.11.2004**
(21) Application number: 03701451.1
(22) Date of filing: 15.01.2003
(51) Int. Cl.: C08G 65/00

(54) **MULTIDROP TREE BRANCHING FUNCTIONAL POLYETHYLENE GLYCOL, THE PREPARATION METHOD AND THE USE OF IT**

(30) Priority: 15.01.2002 CN 02101672
(71) Applicant: Pan Asia Bio (Shanghai) Co., Ltd, Shanghai 201203 (CN)
(72) Inventor: HUANG, Junlian, Shanghai 201203 (CN)
(74) Representative: Gleiss, Alf-Olav, Dr.jur. Dipl.-Ing.
(86) International application number: PCT/CN2003/000029
(87) International publication number: WO 2003/059987

(57) **Abstract**

The present invention designs and synthesizes a kind of new Multidrop tree branching functional polyethylene glycol with active end group, can be extensive used in modify of small molecular medicines, proteins and polypeptide medicines, used in improving solubility, stability and immunological competence in order to prolong medicines half-life period, increase curative effect.

## Description

### FIELD OF THE INVENTION

This invention relates to the design and synthesis of polymers or macromolecules. In details, the synthesis methods of the multiple branched, functional polyethylene glycols from a series chemical reaction of linear polyethylene glycols and small molecules are disclosed.

### BACKGROUND OF THE INVENTION

There are various types of pharmaceutical drugs (including anti-tumor drugs) which can be classified generally into two categories based on their molecular weights: Type one drugs are molecules with relatively lower molecular weights, whose molecular weight are usually smaller than 1000 Da. Most of this type of drugs are chemically synthesized molecules or laborious extraction and purification from natural sources. Such as nitrogen mustards, cis-platinum, 5-fluorouracil, as well as paclitaxel etc. belong to this category; Another type is the macromolecule drugs, most of which are genetically engineered recombinant proteins or peptides drugs. Both of two kinds of drugs are worrisome due to higher toxicity, poor solubility, and relatively lower half life. Furthermore, most of proteins and peptides drugs are immunogenic, which can induce the harmful immunoreactions.

One of the methods to resolve those problems is through the chemical modification of the drugs with polymers. Caliceti, T. et al. [J. Bioactive Compatible Polym. 10:103-120 (1995)] reports the modification of superoxide dismutase with polyvinyl pyrrolidone. Uren, J. R. et al. (Cancer Res. V39, 1927-1933, 1981) discusses the modification of L-asparaginase with polyDL-alanine . Among the literatures and patents, the mostly popular chemical modification method is using activated polyethylene glycol (PEG) as a matrix of drugs Nandini, K et al. (Eup. Pat. 87304703.9) and Mike. A. et al. (PCT/US 98/00683) describes the reaction of tumor necrosis factor (TNF) with PEG; Gilbert, C. W et a.l. (US patent 5951974, US Patent 5981709 and US Patent 6042822) reports that using PEG to chemically modify interferon- . Similar reactions such as modification of interleukin-2 with PEG (Prakash, R. K. el. al US Patent 6251866), modification of granulocyte-macrophage colony-stimulating factor (GM-CSF) with PEG [Knusli, C el at Br. J. Haematl, v82 (4), 654-663(1992); Malik, F et al Exp. Hemaol, v20(8), 1028-1035(1992)], modification of growth hormone with PEG, are reported in many literatures and patents.

In the chemical reactions of PEG modification of pharmaceuticals, the commonly used active groups are succinimide active esters, aldehyde group, trifluorsulfonate, p-nitrophenylcarbonate, benzotriazole carbonate etc. In terms of structure of PEG molecules, the commonly used PEG derivatives are either linear monoemethoxypoly ethylene glycol or branched one with molecular weight ranging from 2,000 Da to 60,000 Da (Monfardini, C. et al. Bioconjugate Chem. 6:62-69,1995).

It is well known that the properties of modified pharmaceuticals by polymers depend on the structure, the molecular weight, the molecular weight distribution, as well as the configuration of polymer materials used. Therefore the properties of a drug modified by a PEG derivative with the same structure but different molecular weights are different. Furthermore, there are differences among the properties of drugs modified with the same structure and the same molecular weight but different configuration of PEG polymers. Previously, some difficulties have impeded the benefits of chemical modification of proteins or other pharmaceuticals with PEG. One of these difficulties is lack of conjugating selectivity, which leads to conjugation at active sites of the proteins, therefore the bioactivity of drugs (like proteins) will be reduced subsequently. Another difficulty is the site specific conjugation. For example, some proteins have multiple sites that can be modified by PEG molecules. It is difficult for the PEG modification at the specific site of these proteins. Consequently, it is difficult for quality control for these pharmaceutical conjugates.

### DETAILS OF THE INVENTION

The purpose of this invention is to design and synthesize a series of multiple branched polyethylene glycols (PEGs) polymers with differently active and functional terminal groups. Compared to linear and branched polyethylene glycol (PEGs) polymers with the same molecular weights, this new kind of multiple branched (or multiple armed) PEG polymers have several benefits/advantages: they have lower static viscosity and greater hydrodynamic volume. Therefore, the pharmaceuticals (protein drugs and small molecule drugs) modified by this new kind of multiple branched PEG polymers will have better physical and chemical properties.

Through a series of the reactions of small molecules with tri-functional groups, we synthesize a new kind of PEG polymers using a number of linear PEG polymers of certain molecular weights. This new kind of PEG polymers are structurally multiple branched (multiple armed) and functional that active groups attached at the terminal of multiple branched PEGs. The new molecules of polymers of the invention can be represented structurally as:

RPEG_{z}COX.

Wherein
R represents the straight chain alkanes with 1∼10 carbon atoms, isopropyl and benzyl groups;
z represents the numbers of the branches (or arms);
X represents functional groups, which are functional groups capable of bonding with a biologically active nucleophile selected from the group consisting of proteins, peptides, polypeptides, enzymes and chemotherapeutic molecules or a moiety capable of being functionalized to react with said nucleophile.

Linear polyethylene glycols are linked with small molecules with tri-functional groups through covalent bonds. The linking groups between linear polyethylene glycols (PEGs) and small molecules can be amide, imide, carbamate, ester, epoxy, carboxyl, hydroxyl, thiol, carbohydrate and the combinations of these groups. The "tri-functional small molecules" can be one of followings: 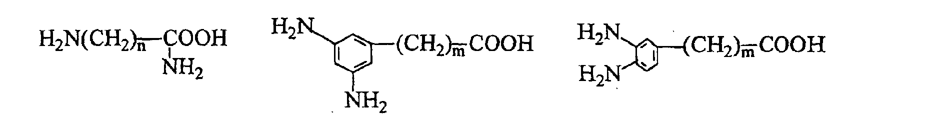 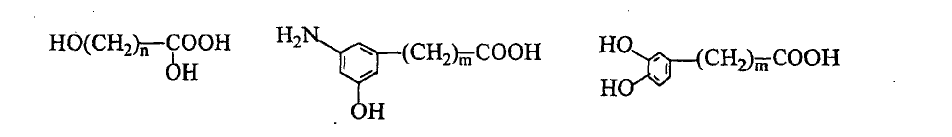 n represents the integer from 1 to 9; m represents the integer form 0 to 6

For example, when the "tri-functional small molecule" is H₂N(CH₂)ₙCH(NH₂)CO₂H (n represents the integer form 1 to 9), and the linking group is amide or carbamate, a new functional PEG polymer with 8 branches (arms) (RPEG₈COX) is synthesized. The structure of functional PEG polymer with 8 (RPEG₈COX) branches is represented as following: 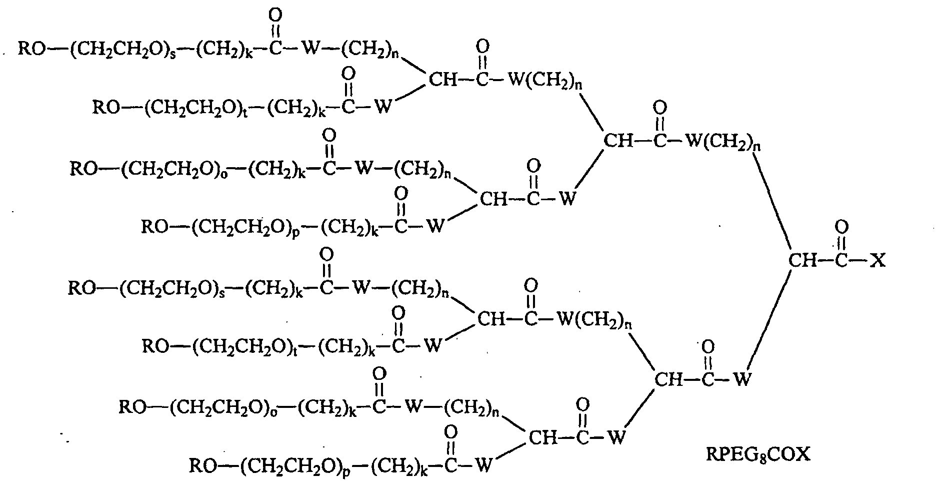 Wherein,
R represents the straight chains alkanes with 1∼10 carbon atoms, isopropyl, or benzyl groups, among them methyl is preferred;
n represents the integer from 1 to 9;
k represents integer from 0 to 6;
s, t, o, p represent the integers from 10 to 2,000;
W is one of O,S or NH groups;
X is one of the followings:

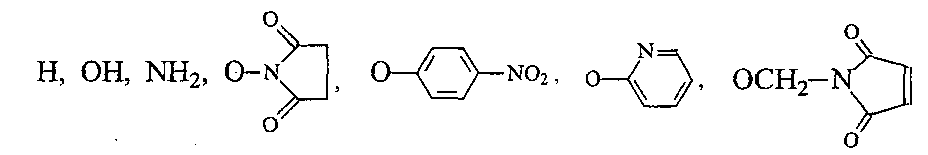

The structures of the other (other than eight branches, e.g. one to seven branches) multiple branched and functional PEG polymers are similar to that of RPEG₈COX. The exact structures of those branched PEG polymers are depending on the numbers of branches. For example, in a structure of a seven branched (7 armed) PEG polymer, a linear PEG molecule will replace (or substitute) one of two branched PEG molecules in a eight branched PEG polymer (RPEG₈COX); in a structure of six branched (6 arms), there are 6 linear PEG polymers (molecules). The rest may be inferred. In this kind of multiple branched (or multiple armed) PEG polymers, the molecular weight of each branch (or arm) ranges from 400 Da to 80,000 Da.

The synthesis methods of a branched PEG polymer have been reported previously by several groups, e.g. Yamsuki et al. (Agric Bio. Chem. 1988, 52, 2185-2196), Monfardini et al. (Bioconjugate Chem. 1995, 6, 62-69). All these publications and literatures are references of this invention.

The functionalization (activation) methods of the multiple branched PEG polymers (three to eights branches PEG polymers) are similar each other. The functionalization (activation) methods can be demonstrated by following chemical reactions of three branches (or arms) and four branches (or arms) PEG polymers, and the activation of linear or branched PEG polymers reported by Monfardini is used as references..

When
W is NH,
n is 4,
k is 0, and
X is p-nitrophenol,

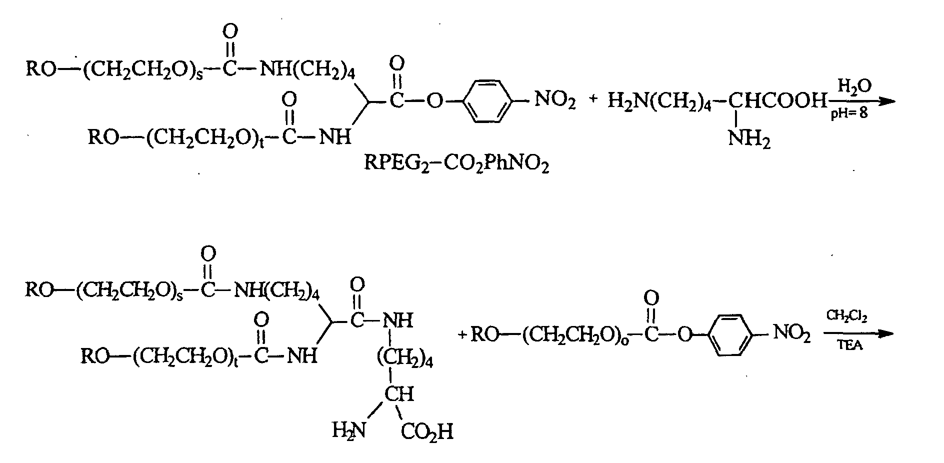

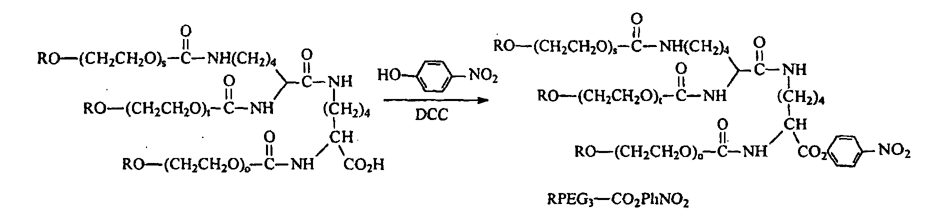

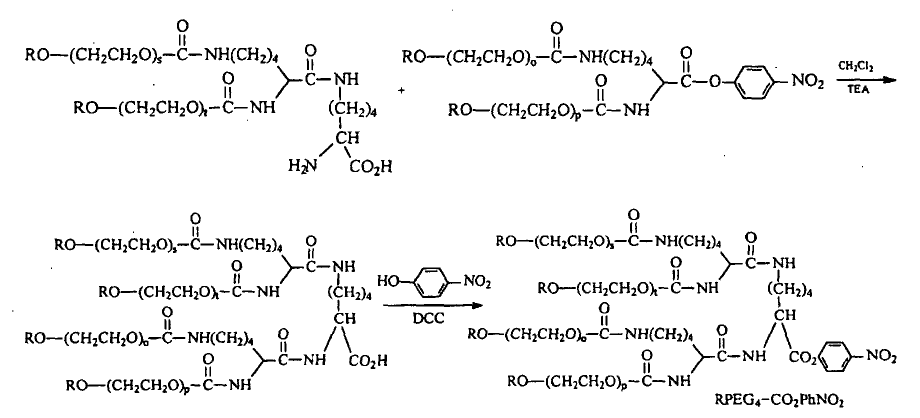

The activation of the carboxylic acid (group) in the chemical reactions or routes above is achieved through p-nitrophenol ester, or through other active ester, such as succinimidyl esters.

In the activation of the carboxyl acid through the succinimdyl ester route, if one amide and one carboxyl of "tri-functional small molecules" are protected in advance, the control of molecular weight of each branch (or arm) PEG polymer can be achieved.

For example, if the carboxyl acid is activated through succinimidyl ester group, the activations of three branches (or three arms) and four branches (or four arms) PEG polymers can be achieved, wherein the (Lys)₂Cu is prepared (or synthesized) according to the methods described by Wiejak's et al. (S. Wiejak, et al CHem. Pharm. Bull. 1999, 47, 1489-1490).

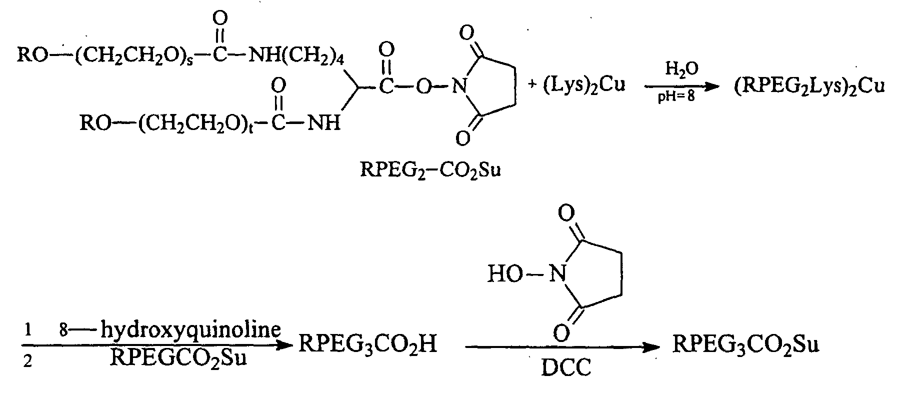

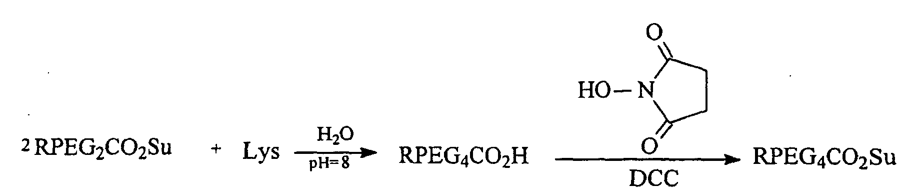

According to the chemical reactions described above, it is easy to synthesize every multiple branched PEG polymer that differ in its numbers of branches (or arms) ranging from three to eight branches as only a small and necessary adjustment of the reaction conditions is made. By publishing this invention, it is obvious and simple processes for the skilled technicians of this field.

The multiple branched and functional PEG polymers of this invention can be used widely in the chemical modifications of small molecule drugs, proteins as well as peptide drugs. For example, the small molecule drugs such as chlorambucil, cis-platinum, 5-fluorouracil, paclitaxel, adriamycin, metrotrexate; protein pharmaceuticals, such as interferon, interleukin, tumor necrosis factor, growth hormone, granulocyte colony-stimulating factor (G-CSF), ethythropoietin (EPO), superoxidedismutase (SOD) can be conjugated or modified by this new type of PEG polymers. The conjugation processes can be followed by the reported methods using linear PEG polymers (Greenwald, et al Bioorg. Med. Chem. Lett. 1994, 4, 2465; Caliceti, et al IL Farmaco, 1993, 48, 919; Zalipsky and Lee, Poly (ethylene glycol) chemistry: biotechnical and biomedical applications. Eddied by J. M. Harris, Pleunm Press, N. Y. 1992). These published literatures are used as references for this invention. With the publication of the invention, it become obvious to the skillful technicians of this field to conjugate various pharmaceuticals or chemicals using this new kind of PEG polymers
The advantages of the invention are as followings:
1) These multiple branched PEG polymers have more hydrodynamic volume than linear or two branched PEG polymers that have the same molecular weight (size). When these multiple branched PEG polymers are used to modify (surface of) drugs or proteins at a site, then adjacent sites at the drugs or (the surface) proteins will be difficult to react with another such PEG polymer due to a larger and steric effect of this kind of PEG polymers. Therefore the selectivity of the conjugation by this type new PEG polymers is improved compared to linear PEG or two branched one with the same molecular weight.
2) By controlling and changing the molecular weight of each branch PEG molecule and the number of the arms of PEG polymers, the hydrodynamic volume of PEG polymer can be controlled and adjusted, which makes difficult for these PEG polymers to approach to the active site of the protein. Therefore, the bioactivity of these PEG-protein conjugates will be preserved at a relatively high level.
3) Compared to the linear or branched PEG polymer, the structure of multiple branched PEG polymers can hinder efficiently the macromolecules and the cells to approach the protein conjugates. So that they will improve the circulating time of the PEG-protein conjugates and reduces the immunoreactions of the proteins.

### EXAMPLES

The invention is further illustrated in more detail by following examples. Such examples are for the illustration purpose but not to limit the invention.

In the following examples,
R is methyl group,
mPEG is monomethoxypoly(ethylene glycol),
GPC is gel penetration chromatography, and
MALDI is matrix-assisted laser desorption/Ionization mass Spectrum.

### Example 1

### The preparation of three branched mPEG₃CO₂PhNO₂ with different lengths of branches (the molecular weights of each arm are 7 Kd, 3 Kd and 5 Kd, respectively)

439 mg lysine (3 mmol) was resolved into 20 ml water (pH 8.0 to pH8.3). Then 10g (01 mmol) mPEG₂CO₂PhNO₂, which are two branches (or arms) PEG polymer (MW 10,000 Da) with one branch of MW 7,000 Da and another branch of MW 3,000 Da, was added in batches. pH of the solution was adjusted to 8.3 with 0.2 N NaOH. The mixture solution was stirred at room temperature for overnight and then was cooled down to 0 . The pH of the reaction products was then adjusted to pH3 with 2N HCl. The impurities were extracted with ether at first and then the residues were extracted three times with chloroform. The extraction was concentrated, in which ether was added, and white precipitation appeared. The product of mPEG₂-mono-Lysine was then purified by re-crystallization by ethanol. The purity is more than 99% by characterization of amine titration, GPC and MALDI.

The 9g above-mentioned mPEG₂-mono-Lysine (0.9mmol) from above reactions was resolved in 20ml dried dicholormethane, and the pH of the solution was adjusted to pH8 with triethylamine (TEA). 5.025g mPEGCO₂PhNO₂ (1.05mmol, a linear PEG of MW 5,000 Da) was added into the solution in batches within 3 hours, and the solution pH was maintained at about 8 with TEA. The solution was refluxed for 72 hours, and was cooled down to room temperature. The solution was concentrated, then filtrated. The filtrate was poured into ether slowly. Ether was added to precipitate and the mixtures were filtrated, re-crystallized with ethanol. The Excessive mPEGCO₂PhNO₂ was stirred to hydrolyzed in Na₂CO₃ buffer (pH 9∼10) for overnight. The solution was cooled down to 0 , and the pH was adjusted to pH3 with 2N HCl. p-nitrophenol in the solution was extracted with ether. The solution was extracted with chloroform for three times. And the extraction was dried, concentrated and precipitated with dry ether. The product was then re-crystallized with dry ethanol and was purified further with QAE Sephadex A50 column ( 5 × 80cm, elution: borate buffer of pH 8.9) to get final product, mPEG₃CO₂H (the lengths of three arms are 7 Kd, 3 Kd and 5 Kd, respectively). The product was characterized by titration of carboxyl, GPC and MALDI. The purity of the product is more than 99%.

9g (0.6mmol) PEG with three branches (or 3 arms, mPEG₃CO₂H) from above reactions was resolved in 20ml dry dichloromethane, 0.167g p-nitrophenol (1.2mmol) and 0.48g DCC (1.2 mmol) were added into the solution. The solution was stirred at room temperature overnight, then was filtrated. The filtrate was concentrated and precipitated with ether, and the products were re-crystallized with ethyl acetate to get products of mPEG₃CO₂PhNO₂. After hydrolysis of the products in a basic buffer, its purity or content of activated ester (p-nitrophenol anion) was assayed by UV spectrum. The content of activated ester was above 98%.

### Example 2

### Preparation of three branched mPEG₃CO₂Succinimide of different arm lengths (MWs are 7 Kd, 3 Kd, and 5 Kd, respectively)

9g three branched mPEG₃CO₂H (0.6mmol) (each length of three branches are 7 Kd, 3 Kd, and 5 Kd, respectively)of the example 1 above was resolved in 20ml dry dichloromethane, and was cooled down to 0 . 0.138g N-hydroxysuccinimide (1.2 mmol) and 0.48g of DCC (1.2mmol) were then added into the cool solution, and the solution was stirred overnight, and was filtrated. The filtrate was precipitated with ether and re-crystallized with ethyl acetate. The purified product was mPEG₃CO₂Succinimide. The purity of activated ester of the product was assayed with the UV spectrum; the value is greater than 98 %.

### Example 3

### Preparation of a four branched mPEG₄CO₂PhNO₂ (MW 20 Kd) of different arm lengths (MWs are 7 Kd, 3 Kd, 6 Kd and 4 Kd, respectively)

9g mPEG₂-mono-Lysine (0.9mmol) (The MWs of each arm are 7 Kd and 3 Kd, respectively) from example 1 above was solved in 20ml dry dichloromethane, and the pH of the solution was adjusted to 8 with triethylamine (TEA). 10.5g of another two branched mPEG₂CO₂PhNO₂. (1.05mmol, one arm of MW 6,000 Da and another one of MW 4,000 Da) was added into the solution in batches within 3 hours. The pH of the solution was maintained at 8 with TEA. The solution was refluxed for 72 hours, and then was cooled down to room temperature. The reaction mixture was then concentrated, filtrated, and precipitated with ether subsequently. The crude product was re-crystallized with dry ethanol and then purified with QAE Sephadex A50 column ( 5 × 80cm, elution: pH 8.9 borate buffer) to get a product was four branched mPEG₄CO₂H polymer of MW 20 Kd (7 Kd, 3 Kd, 6 Kd and 4 Kd in each branch, respectively). Assayed by carboxyl titration, GPC, MALDI, the purity of four branched PEG molecules is 98.5%.

The activation of this four branched PEG polymer is as following: 12g four branched mPEG₄CO₂H (0.6mmol) was resolved in 25ml dry dichloromethane. 0.167g of p-nitrophenol (1.2mmol) and 0.48g of DCC (1.2mmol) were added into the solution. Then, the solution was stirred overnight, and then filtrated. The filtrate was concentrated and precipitated with ether. The crude product was re-crystallized with ethyl acetate to get pure mPEG₄CO₂PhNO₂. The purity of this four branched mPEG₄CO₂PhNO₂ which war assayed by UV spectrum was greater than 98%.

### Example 4

### Preparation of three branched mPEG₃CO₂PhNO₂ of different arm length (7 Kd, 3 Kd and 5 Kd in each branch) (method 2)

2 mM Lysine hydrochloride (365mg) was added into the 2 M NaHCO₃ solution (2ml) under stirring, then 2 ml of 0.5 M CuSO₄ was added into the solution, and the pH was maintained at 8 with borate buffer. 20 g (2 mM) two branched mPEG₂CO₂Succinimide ester (with one branch of 7 Kd and another of 3 Kd) was introduced into the solution, NaOH was used to maintain the pH of the solution at pH8.0. The solution was stirred for 24 hours at the room temperature and then the reactant was diluted to 100ml with de-ionized water. The pH of the solution was adjusted to 3 with oxalic acid and then extracted with dichloromethane three times. The extract was concentrated and precipitated with dry ether. The white precipitant was re-crystallized twice with ethanol to get (mPEG₂Lys)₂Cu.

20 g (2 mM) (mPEG₂Lys)₂Cu from above was then resolved in 40ml acetone. 4.2 mM of 8-hydoxy quinoline and 20 ml of 10% Na₂CO₃ solution were then added into the solution, and the solution was stirred for 1 hour at room temperature. 10 g (2 mM) of a linear mPEGCO₂Succinimide ester (a linear mPEG succinimide ester with MW 5,000 Da) was added into the solution, and the pH of the solution was maintained at 8.0 with NaOH. The solution was stirred for 4 hours, and filtrated. The pH of the filtrate was then adjusted to 3 with oxalic acid and then extracted with dichloromethane three times. The extract was concentrated, and precipitated with the dry ether. The white precipitation was re-crystallized three times with ethanol to get a product of three branched mPEG₃CO₂H of MW 15 Kd (each of three branches is 7 Kd, 3 Kd and 5 Kd, respectively). The product was then purified with QAE Sephadex A50 column ( 5 × 80cm, elution buffer: pH 8.9 borate buffer) and characterized by titration of carboxyl, GPC and MALDI. The purity of this three branched PEG molecules of MW 15 Kd is more than 99%

The activation of this three branched mPEG₃CO₂H is as following:

9g (0.6 mM) of three branched mPEG₃CO₂H from above was resolved in 20ml dry dichloromethane, and the solution was cooled down to 0 . 0.138g N-hydroxysuccinimide (1.2 mmol) and 0.48g of DCC (1.2mmol) were then added into the cool solution while the solution was stirring for overnight. The solution was filtrated and precipitated with ether. The crude product was re-crystallized with ethyl acetate to get a product of mPEG3CO₂Succinimide. The content of the activated ester of the product was assayed by UV spectrum; the value is more than 98%.

### Example 5

### Preparation of four branched mPEG₄CO₂Succinimide of MW 40 Kd with the same length in each branch (or arm)

2 mM Lysine hydrochloride (365mg) was added into 100 mL borate buffer. 80 g (4 mM) of two branched 20 Kd mPEG₂CO₂Su (10 Kd MW in each branch) was added into the solution while the pH of the solution was maintained at pH8.0 with 0.2 N NaOH. The solution was stirred 24 hours at room temperature and was diluted to 400ml de-ionized water. The pH of the solution was adjusted to 3 with oxalic acid. Then the solution was extracted with dichloromethane three times. The extract was concentrated and precipitated with dry ether. The white precipitant was re-crystallized twice with ethanol to get a product of crude four branched mPEG₄CO₂H. The crude product was then purified with DEAE Sephadex FF column to get purified four branched mPEG₄CO₂H. The yield of the purification of this four branched (with each branch of MW 10 Kd) is 92%. The purified product was confirmed by a series of assays such as NMR, GPC and MALDI spectrum.

The activation of this four branched PEG molecules is as following:

24g (0.6 mM) of purified four branched mPEG₄CO₂H from above was resolved in the 20ml dry dichloromethane, and was then cooled down to 0 . 0.138g (1.2 mM) N-hydroxysuccinimide and 0.48g (1.2 mM) DCC were added into the cool solution. The solution was stirred overnight, and filtrated. The filtrate was precipitated with ether, and re-crystallized with ethyl acetate to get pure four branched mPEG₄CO₂Succinimide ester. The content of activated ester in the product was assayed by UV spectrum, and the value is greater than 96%.

### Example 6

### Preparation of Eight branched (80 Kd) mPEG₈CO₂Succinimide of the same arm length (each branch of 10 Kd in length)

The eight branched (of total MW 80 Kd) mPEG₈CO₂Su was produced by the method of described in the Example 5 above using four branched (40 Kd MW with each branch of 10 Kd) mPEG₄CO₂Su, and the final yield of eight branched mPEG₈CO₂Su was 85%.

In the following examples, we demonstrated the synthesis of multiple branched PEG molecules with the same length of each branch using PEG molecules with the same length branches as well as the synthesis of multiple branched PEG molecules with different length branches using PEG molecules with different length branches.

### Example 7

### Preparation of five branched mPEG₅CO₂Su and mPEG₅CO₂PhNO₂

The five branched mPEG₅CO₂PhNO₂ was produced using the method described in Example 1 above. Three branched mPEG₃CO₂PhNO₂ and two branched mPEG₂CO₂PhNO₂ were used as material. The final yield of five branched mPEG₅CO₂PhNO₂ is 90%.

The five branched mPEG₅CO₂Su was produced using the method described in Example 2 above, and three branched mPEG₅CO₂Su and two branched mPEG₅CO₂Su were used as material. The final yield of five branched mPEG₅CO₂Su is 91%.

### Example 8

### Preparation of six branched mPEG₆CO₂Su and mPEG₆CO₂PhNO₂

Six branched mPEG₆CO₂PhNO₂ was produced using the method described in Example 1 above. Three branched mPEG₃CO₂PhNO₂ were used as material. The final yield of six branched mPEG₆CO₂PhNO₂ is 89%. Alternatively, six branched mPEG₆CO₂PhNO₂ was produced using the method described in Example 1 using two branched mPEG₂CO₂PhNO₂ and four branched mPEG₄CO₂PhNO₂ subsequently as material. The final yield of six branched mPEG₆CO₂PhNO₂ is 92%.

The six branched mPEG₆CO₂Su was produced using the method described in the Example 2 above, and the final yield of six branched mPEG₆CO₂Su is 88%.

### Example 9

### Preparation of Seven branched mPEG₇CO₂Su and mPEG₇CO₂PhNO₂

The seven branched mPEG₇CO₂PhNO₂ was produced using the method described in the Example 1 using three branched mPEG₃CO₂PhNO₂ and two branched mPEG₄CO₂PhNO₂ as materials. The final yield of seven branched mPEG₇CO₂PhNO₂ was 86%.

The seven branched mPEG₇CO₂Su was produced using the method described in the Example 2, and the final yield of seven branched mPEG₇CO₂Su was 87%.

### Example 10

### Preparation of Eight branched mPEG₈CO₂Su and mPEG₈CO₂PhNO₂

The eight branched mPEG₈CO₂PhNO₂ was produced by the method described in the Example 1 using three branched mPEG₃CO₂PhNO₂ and two branched mPEG₅CO₂PhNO₂ as material. The yield of eight branched mPEG₈CO₂PhNO₂ was 80%. Alternatively, eight branched mPEG₈CO₂PhNO₂ was produced by the method described in the Example 1 using four branched mPEG₄CO₂PhNO₂ as material. The final yield of eight branched mPEG₈CO₂PhNO₂ was 79%.

Eight branched mPEG₈CO₂Su was produced by the method described in the Example 2, and the yield of eight branched mPEG₈CO₂Su was 78%.

### Example 11

The multiple branched (3 arms, 4 arms, 5 arms, 6 arms, 7 arms, and 8 arms) and functional PEG polymers with aldehyde groups were produced by the methods described in the Examples 1 to 10.

### Example 12

The multiple branched (3 arms, 4 arms, 5 arms, 6 arms, 7 arms, and 8 arms) and functional PEG polymers with carboxyl groups were produced by the methods described in the Examples 1 to 10.

### Example 13

The multiple branched (3 arms, 4 arms, 5 arms, 6 arms, 7 arms, and 8 arms) and functional PEG polymers with amino groups were produced by the methods described in the examples 1 to 10.

### Example 14

The multiple branched (3 arms, 4 arms, 5 arms, 6 arms, 7 arms, and 8 arms) and functional PEG polymers with carboxyl groups were produced by the methods described in the examples 1 to 10.

### Example 15

The multiple branched (3 arms, 4 arms, 5 arms, 6 arms, 7 arms, and 8 arms) and functional PEG polymers with succinimide groups were produced by the methods described in the examples 1 to 10.

## Claims

1. The multiple branched (or armed) and functional polyethylene glycol comprising the formula or the structure:
RPEG_{z}COX;
Wherein:
R is a straight-chain (or linear) alkane containing C₁ C₁₀. of carbons groups, isopropyl or benzyl group, among them the methyl group is preferred;
z is the numbers of PEG polymer branches (or arms);
X is the functional group.

2. A multiple branched, functional polyethylene glycol, wherein said polyethylene glycol according to claim 1 wherein if R is a straight-chain (or linear) alkane containing C₁ C₁₀ of carbons groups, isopropyl or benzyl group, Z is the number of the arms and is from 1 to 8; wherein X is represented by the structure:

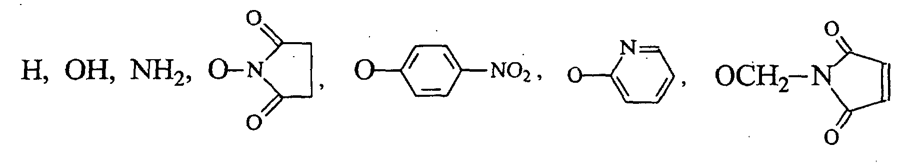

3. A multiple branched (eight arms) and functional polyethylene glycol polymer (RPEG₈CO₂X) is represented one of multiple branched and functional polyethylene glycol polymers of claim 1; wherein the tri-functional small molecular of RPEG₈COX is represented by the structure of 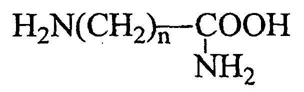 Wherein, n is the integer from 1 to 9;
wherein said this multiple branched and functional polyethylene glycol polymer is represented and demonstrated in following the structure: 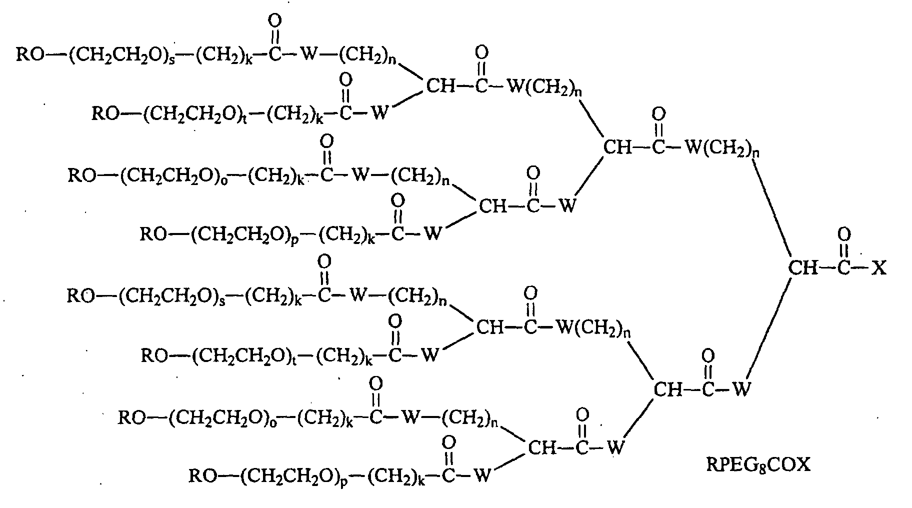 Wherein:
R is a straight-chain (or linear) alkanes containing C₁ C₁₀ of carbons groups, isopropyl or benzyl groups, among them methyl group is preferred;
n is a positive integer from 1 to 9;
k is a positive integer from 1 to 6;
s, t, o, and p are positive integers from 10 to 2,000;
W is one of following groups: O, or S, or NH;
X is selected from one of the following groups.

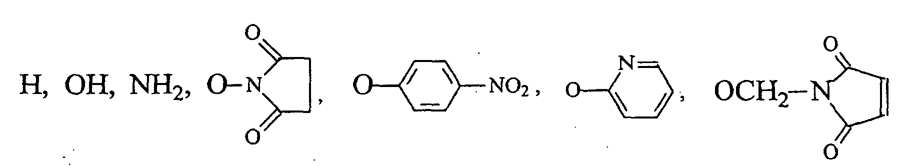

4. A process of preparing the multiple branched and functional polyethylene glycol polymers of claim 1, wherein said multiple branched and functional polyethylene glycol polymers of RPEG₂COX is produced through a series of chemical reactions using straight-chain or linear polyethylene glycol polymers and tri-functional small molecules.

5. A process of preparing the multiple branched and functional polyethylene glycol polymers of claim 4, wherein the active groups of a tri-functional small molecule are one of following combinational groups: one carboxyl group and two amino groups, or one carboxyl group and two hydroxyl groups.

6. A process of preparing the multiple branched and functional polyethylene glycol polymers of claim 4, wherein said the multiple branched and functional polyethylene glycol polymers are synthesized through a series of chemical reactions using straight-chain or linear polyethylene glycol polymers and the tri-functional small molecules. The structures of the "tri-functional small molecules" are represented as following: 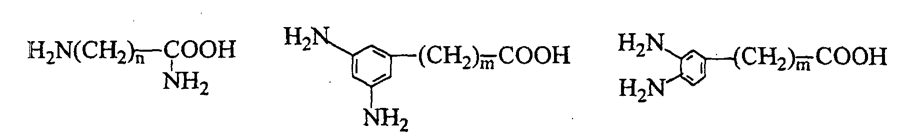 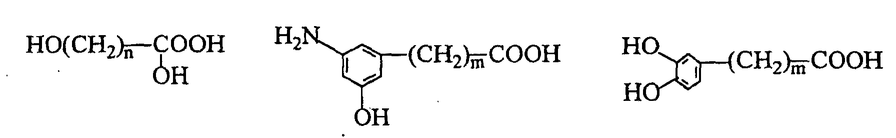 wherein:
n is a positive integer from 1 to 9;
m is a positive integer from 0 to 6.

7. A process of preparing the multiple branched and functional polyethylene glycols of claim 4, wherein said the multiple branched and functional polyethylene glycol polymers are produced by a series of chemical reactions using straight-chain or linear polyethylene glycol molecules and tri-functional small molecules; wherein the molecular weight of each branch or arm of these straight-chain or linear polyethylene glycol polymers is ranging from 400 Da to 80,000 Da; wherein the multiple branched and functional polyethylene glycol polymers are polymers with 3 arms, or 4 arms, or 5 arms, or 6 arms, or 7 arms, or 8 arms, respectively.

8. A process of preparing the multiple branched and functional polyethylene glycol polymers of claim 4, wherein said the multiple branched and functional polyethylene glycol polymers are produced through a series of chemical reactions using straight-chain or linear polyethylene glycol polymers and tri-functional small molecules; wherein the linking groups between polyethylene glycol and tri-functional small molecules are:
amide, imide, carbamate, ester, epoxy, carboxyl, hydroxyl, thiol, carbohydrate or the combinations of these groups.

9. A process of preparing conjugates using the multiple branched and functional polyethylene glycol polymers of claim 1, wherein said the conjugates are forming through a covalent bond between a functional group on these multiple branched polyethylene glycol polymers and biologically active molecules; wherein the biologically active molecules are small molecule drugs, peptides, monoclonal antibody or protein drugs.

10. A process of preparing conjugates using the multiple branched and functional polyethylene glycol polymers of claim 9, wherein said small molecule drugs are chlorambucil, cis-platinum, 5-fluorouracil, taxol, adriamycin, metrotrexate.

11. A process of preparing conjugates using the multiple branched and functional polyethylene glycol polymers of claim 9, wherein said the biologically active molecules are proteins such as interferons, interleukins, tumor necrosis factor, growth hormone, granulocyte colony-stimulating factor, ethythropoietin (EPO), superoxidedismutase (SOD) etc.
